(19) Europäisches Patentamt
European Patent Office
Office européen des brevets

(11) Numéro de publication : **0 107 599**
**B1**

# (12) FASCICULE DE BREVET EUROPÉEN

(45) Date de publication du fascicule du brevet : **11.12.85**

(51) Int. Cl.⁴ : **C 07 C 69/44**, C 07 C 67/38

(21) Numéro de dépôt : **83420164.2**

(22) Date de dépôt : **17.10.83**

## (54) Procédé de préparation d'esters linéaires saturés par carbonylation de composés monooléfiniques.

(30) Priorité : **19.10.82 FR 8217663**

(43) Date de publication de la demande :
**02.05.84 Bulletin 84/18**

(45) Mention de la délivrance du brevet :
**11.12.85 Bulletin 85/50**

(84) Etats contractants désignés :
**AT BE CH DE FR GB IT LI LU NL SE**

(56) Documents cités :
**FR-A- 2 091 775**
**BULLETIN OF THE CHEMICAL SOCIETY OF JAPAN, vol. 46, no. 2, 1973, pages 524-530, The Chemical Society of Japan, Tokyo, JP. A. MATSUDA: "The cobalt carbonyl-catalyzed hydroesterification of butadiene with carbon monoxide and methanol"**

(73) Titulaire : **RHONE-POULENC CHIMIE DE BASE**
**25, quai Paul Doumer**
**F-92408 Courbevoie (FR)**

(72) Inventeur : **Jenck, Jean**
**4, Avenue de la Paix**
**F-68490 Chalampe (FR)**

(74) Mandataire : **Varnière-Grange, Monique et al**
**RHONE-POULENC RECHERCHES Service Brevets Chimie et Polymères Centre de Recherches de Saint-Fons 85, avenue des Frères Perret B.P. 62**
**F-69192 St-Fons Cédex (FR)**

**EP 0 107 599 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un procédé de préparation d'esters linéaires saturés par carbonylation de composés monooléfiniques, c'est-à-dire par réaction du monoxyde de carbone et d'un alcool sur des composés renfermant une liaison oléfinique unique.

L'invention a plus spécifiquement pour objet la préparation d'adipates d'alkyles par carbonylation des penténoates d'alkyles.

Il est bien connu, d'après le « Bulletin of the Chemical Society of Japan, vol. 46, 1973, pages 526 et 527 », qu'on obtient un mélange renfermant des diesters d'alkyle et notamment, un adipate d'alkyle, en faisant réagir du monoxyde de carbone et un alcool sur un pentène-3 oate d'alkyle, sous haute pression et à température élevée, en présence de cobalt carbonyle et d'une base azotée hétérocyclique et aromatique. Cependant le développement à l'échelle industrielle d'une telle technique, dont l'intérêt de principe n'est pas contesté, est largement compromis, non seulement par la faible efficacité du système catalytique, mais aussi par la proportion notable de pentanoate d'alkyle formée, bien que la réaction soit effectuée en l'absence d'hydrogène.

Il est par ailleurs bien connu des spécialistes de ce domaine que la présence de faibles quantités d'hydrogène dans le milieu réactionnel tend à augmenter l'efficacité des catalyseurs à base de cobalt, dans les procédés de synthèse d'esters par réaction d'un alcool et du monoxyde de carbone sur un composé oléfinique.

La Demanderesse a néanmoins constaté que dans la plupart des cas cet effet favorable, lié à la présence de faibles quantités d'hydrogène, est accompagné par une influence négative sur la sélectivité en esters linéaires, produits spécifiquement visés.

En effet, on observe que la présence d'hydrogène non seulement tend à augmenter la proportion de produits d'hydrogénation dans le mélange réactionnel, mais aussi elle est susceptible de diminuer la proportion d'ester linéaire parmi les esters formés.

Cet effet négatif grève lourdement l'économie de ces procédés dans la mesure où la valorisation des esters branchés et des produits d'hydrogénation est aléatoire, voire inexistante. Tel est notamment le cas des diesters branchés et des pentanoates d'alkyles produits lors de la carbonylation de penténoates d'alkyles. En effet ces produits étant en pratique détruits, leur formation correspond, en d'autres termes, à une perte intolérable de matière première. Par ailleurs de l'hydrogène peut être formé in situ à partir des traces d'eau susceptibles d'être contenues par des réactifs de qualité technique, selon la réaction bien connue :

$$H_2O + CO \longrightarrow CO_2 + H_2$$

Il serait souhaitable, pour des raisons économiques évidentes, de pouvoir utiliser du monoxyde de carbone de qualité technique renfermant de l'hydrogène, sans que cela se produise au détriment de la sélectivité en esters linéaires, esters visés. Il serait également souhaitable, pour les mêmes raisons, de pouvoir utiliser des réactifs renfermant des traces d'eau, sans que cela conduise à une perte de matière première.

Il a maintenant été trouvé de manière tout à fait surprenante qu'il est possible de préparer sélectivement des esters linéaires saturés par réaction d'un alcool de formule R—OH et du monoxyde de carbone sur un composé renfermant une liaison oléfinique unique de formule $R_1CH = CHR_2$ dans lesquelles :

$R_1$ et $R_2$, identiques ou différents, représentent l'hydrogène ou un radical alkyle ayant au maximum 20 atomes de carbone, pouvant être substitué par 1 ou 2 atomes de chlore ou groupes alcoxy renfermant au maximum 4 atomes de carbone,

$R_1$ pouvant en outre représenter un radical $—(CH_2)_p—COOH$, $—(CH_2)_p—COOR_3$ ou $—(CH_2)_p—CN$ dans lequel p est un entier au plus égal à 6 pouvant être nul, $R_3$ représente un radical alkyle comportant au maximum 12 atomes de carbone, un à deux groupements méthylène pouvant en outre comporter un substituant alkyle ayant au maximum 4 atomes de carbone,

$R_1$ et $R_2$ pouvant en outre former ensemble un radical unique bivalent $—(CH_2)_q—$, comportant le cas échéant un ou deux substituants alkyle ayant au maximum 4 atomes de carbone, q étant un entier compris entre 3 et 6 inclus,

et R est un radical alkyle comportant au maximum 12 atomes de carbone éventuellement substitué par un ou deux groupes hydroxyles, un radical cycloalkyle ayant de 5 à 7 atomes de carbone, un radical aralkyle ayant de 7 à 12 atomes de carbone ou un radical phényle, en présence de cobalt, d'une base azotée tertiaire à condition de conduire la réaction dans un solvant choisi parmi les composés hétérocycliques renfermant un seul hétérocycle ; ledit hétérocycle, formé de 5 ou 6 chaînons, contient de 1 à 3 hétéroatomes non adjacents, identiques ou différents et, choisis parmi l'oxygène et le soufre divalent et peut comporter, le cas échéant, un ou deux doubles liaisons carbone-carbone ; un cycle à 5 chaînons pouvant comporter en outre un hétéroatomes d'azote, séparé d'un hétéroatome d'oxygène ou de soufre par au moins un atome de carbone du cycle et relié à l'un des atomes de carbone adjacent par une double liaison.

Selon le présent procédé on fait donc réagir du monoxyde de carbone et un alcool de formule R—OH sur un composé de formule $R_1CH = CHR_2$, dans lesquelles R, $R_1$ et $R_2$ ont la signification donnée ci-avant.

Les matières de départ susceptibles d'être carbonylées selon le présent procédé sont donc des composés renfermant une liaison oléfinique unique interne ou terminale ; ces composés comportent plus spécifiquement de 3 à 20 atomes de carbone.

Par la mise en œuvre du présent procédé on obtient des esters saturés, c'est-à-dire des composés qui renferment d'une part un groupement carboxylate (—COOR) et, d'autre part un atome d'hydrogène de plus que la matière de départ. Parmi ces esters prédomine le composé dont le groupement carboxylate (—COOR) est situé en position terminale sur la chaîne principale de la matière de départ.

Une première catégorie de matières de départ plus particulièrement adaptées a pour formule :

$$R_1CH = CHR_2$$

dans laquelle $R_1$ et $R_2$, identiques ou différents représentent l'hydrogène ou un radical alkyle ayant au maximum 10 atomes de carbone, ou bien forment ensemble un radical unique bivalent $-(CH_2)_q-$ q ayant la signification précédente, ledit radical pouvant comporter le cas échéant 1 ou 2 substituants méthyle. A titre d'exemples de tels composés on peut citer le propylène, le butène-1, le butène-2, les hexènes, les octènes et le dodécène-1.

Une seconde catégorie de matières de départ plus particulièrement appropriées est constituée par les composés de formule :

$$R_1CH = CHR_2$$

dans laquelle $R_1$ représente un radical $-(CH_2)p-COOR_3$, p et $R_3$ ayant la signification précédente, un à deux groupements méthylène pouvant comporter un substituant alkyle ayant au maximum 4 atomes de carbone,

et $R_2$ représente l'hydrogène ou un radical alkyle ayant au maximum 4 atomes de carbone.

Parmi les composés de ce type les penténoates d'alkyles revêtent un intérêt tout particulier, car ils permettent d'accéder aux adipates d'alkyles, intermédiaires de l'acide adipique.

Le présent procédé nécessite la mise en œuvre d'un alcool de formule ROH, R ayant la signification donnée précédemment.

A titre d'exemples d'alcools utilisables dans le cadre du présent procédé on peut citer le méthanol, l'éthanol, l'isopropanol, le n-propanol, le tertio butanol, le n-hexanol, le cyclohexanol, l'éthyl-2 hexanol-1, le dodécanol-1, l'éthylène glycol, l'hexanediol-1,6, l'alcool benzylique, l'alcool phényléthylique et le phénol.

On utilise de préférence un alcanol ayant au maximum 4 atomes de carbone ; le méthanol et l'éthanol conviennent bien à la mise en œuvre du présent procédé.

On peut engager l'alcool et le composé monooléfinique en quantités stœchiométriques. Cependant on utilise de préférence un excès d'alcool, dans la proportion de 1 à 10, ou encore mieux, de 2 à 5 moles d'alcool par mole de composé monooléfinique.

La réaction est conduite en présence de cobalt. N'importe quelle source de cobalt susceptible de réagir avec le monoxyde de carbone dans le milieu réactionnel pour donner in situ des complexes carbonyles du cobalt peut être utilisée dans le cadre du présent procédé.

Les sources typiques de cobalt sont par exemple le cobalt métallique finement divisé, des sels inorganiques tels que le nitrate ou le carbonate de cobalt, des sels organiques en particulier des carboxylates. Peuvent également être employés les cobalt-carbonyles ou hydrocarbonyles ; le dicobaltoctacarbonyle convient bien à la mise en œuvre du présent procédé.

Le rapport molaire entre le composé monooléfinique et le cobalt est en général compris entre 10 et 1 000. Ce rapport est avantageusement fixé à une valeur comprise entre 20 et 300.

Le procédé selon la présente invention nécessite également la présence d'une base azotée tertiaire dont le $pK_a$ est compris entre 3 et 10.

La demanderesse préconise l'utilisation d'hétérocycles azotés formés de 5 à 6 chaînons, pouvant comporter un ou deux substituants choisis parmi les groupes alkyles ou alcoxy ayant au maximum 4 atomes de carbone, le groupe hydroxy et les atomes d'halogène, renfermant éventuellement 2 ou 3 doubles liaisons, et pouvant, par ailleurs, le cas échéant être soudés à un noyau benzénique, sous réserve que les maillons adjacents à l'hétéroatome d'azote ne soient ni substitués, ni communs à deux cycles.

Conviennent plus particulièrement à la mise en œuvre du présent procédé les hétérocycles azotés à 6 chaînons, de $pK_a$ compris entre 4 et 7, notamment la pyridine, la picoline-4, l'isoquinoléine et la lutidine-3,5.

La quantité de base azotée tertiaire utilisée est en général telle que le rapport molaire N/Co soit compris entre 1 et 50. Pour une bonne mise en œuvre de l'invention la demanderesse préconise de fixer ce rapport à une valeur comprise entre 2 et 25.

L'une des caractéristiques essentielles du présent procédé réside dans l'utilisation d'un solvant

choisi parmi les composés hétérocycliques renfermant un seul hétérocycle ; ledit hétérocycle, formé de 5 ou 6 chaînons, contient de 1 à 3 hétéroatomes non adjacents, identiques ou différents et, choisis parmi l'oxygène et le soufre divalent et peut comporter, le cas échéant, une ou deux doubles liaisons carbone-carbone ; un cycle à 5 chaînons pouvant comporter en outre un hétéroatome d'azote, séparé d'un hétéroatome d'oxygène ou de soufre par au moins un atome de carbone du cycle et relié à l'un des atomes de carbone adjacent par une double liaison.

Bien entendu, l'hétérocycle peut comporter de 1 à 4, de préférence 1 ou 2 atomes de carbone substitués par 1 ou 2 groupements qui n'interfèrent pas avec les autres composants du milieu réactionnel et dont la dimension est suffisamment faible pour ne pas trop encombrer stériquement le (ou les) héréatome(s) du cycle. Des substituants admissibles répondent à la formule R'—Y— dans laquelle Y représente le lien valentiel, un atome d'oxygène, un atome de soufre ou un groupe carbonyle et R représente un radical alkyle, aralkyle ou aryle comportant au maximum 10 atomes de carbone.

De même une ou deux paires d'atomes de carbone adjacents de l'hétérocycle peuvent être incluses chacune dans un noyau benzénique ou naphténique accolé à l'hétérocycle.

A titres d'exemples de composés hétérocycliques susceptibles de convenir à la mise en œuvre de la présente invention on peut citer : le tétrahydrofuranne, le méthyl-2 tétrahydrofuranne, l'éthyl-2 tétrahydrofuranne, le phényl-2 tétrahydrofuranne, le dihydro-2,3 benzofuranne, le furanne, le méthyl-2 furanne, l'éthyl-2 furanne, le phényl-2 furanne, le benzofuranne, le dibenzofuranne, le tétrahydrothiophène, le méthyl-2 tétrahydrothiophène, le phényl-2 tétrahydrothiophène, le dihydro-2,3 benzothiophène, le thiophène, le méthyl-2 thiophène, le phényl-2 thiophène, le benzothiophène, le naphtothiophène, le dibenzothiophène, le benzodioxole, le benzodithiolanne-1,3, le benzoxathiolanne-1,3, l'oxazole, le diméthyl-2,5 oxazole, le diphényl-2,5 oxazole, le benzoxazole, le thiazole, le diphényl-2,4 thiazole, le benzothiazole, le tétrahydropyranne, le chromane, l'isochromane, le xanthène, le dioxanne-1,3, le dioxanne-1,4, le benzodioxanne-1,4, le trioxanne-1,3,5, le triméthyl-2,4,6 trioxanne-1,3,5, le thianne, le dithianne-1,3, le dithianne-1,4, le benzodithianne-1,4, le thianthrène, l'oxa-1 thianne-3, l'oxa-1 thianne-4, la phénoxathiine et le trithianne-1,3,5.

De préférence, on fera appel à un composé hétérocyclique non substitué, dans la mesure où l'influence bénéfique observée semble diminuer avec le degré d'encombrement du (ou des) hétéroatome(s) du cycle.

Une première catégorie de composés hétérocycliques convenant particulièrement bien à la mise en œuvre de la présente invention est constituée par les composés hétérocycliques qui renferment exclusivement un (ou plusieurs) hétéroatome(s) de soufre.

Une seconde catégorie de composés hétérocycliques convenant particulièrement bien à la mise en œuvre du procédé est constituée par les composés hétérocycliques qui comportent 6 chaînons et au moins 2 hétéroatomes, de même nature et choisis parmi l'oxygène et le soufre divalent.

Pour une bonne mise en œuvre de la présente invention le composé hétérocyclique (solvant de la réaction) sera choisi dans le groupe constitué par le thiophène, le tétrahydrothiophène, le furanne, le tétrahydrofuranne, le dioxanne-1,4, le dithiane-1,4 et le trithiane.

La quantité de solvant qui a une influence sur la sélectivité de la réaction sera en général supérieure à 20 % (en poids) du mélange réactionnel initial et, pour une bonne mise en œuvre du présent procédé, elle sera comprise entre 30 et 60 % (en poids) dudit mélange.

Selon une variante avantageuse du présent procédé, la réaction est également conduite en présence d'hydrogène. Dans le cadre de cette variante, l'hydrogène représentera au moins 0,1 % (en volume) du monoxyde de carbone, sans dépasser toutefois 3 % (en volume). De préférence, la teneur en hydrogène représentera de 0,2 à 2 % (en volume) du monoxyde de carbone.

Bien entendu, si l'hydrogène peut être introduit dans le milieu réactionnel, de manière commode, sous forme d'un mélange avec le monoxyde de carbone, il peut être aussi alimenté séparément.

La réaction est conduite en phase liquide à une température supérieure à 120 °C, sans qu'il soit utile de dépasser 200 °C, sous une pression de monoxyde de carbone d'au moins 50 bars et pouvant atteindre 1 000 bars. On préfère opérer à une température de l'ordre de 130 à 180 °C et sous une pression de monoxyde de carbone de l'ordre de 100 à 300 bars.

Bien entendu, les conditions de pression et de température optimales seront d'autant plus sévères que la matière de départ sera moins réactive, ce qui se produit, notamment, lorsque le degré de protection stérique de la double liaison augmente.

Le monoxyde de carbone utilisé peut renfermer, en plus de l'hydrogène, des impuretés telles que du dioxyde de carbone, du méthane et de l'azote.

Comme cela a été indiqué en tête du présent mémoire, le procédé selon la présente invention trouve une application plus particulièrement intéressante dans la synthèse de diesters à partir de penténoates d'alkyles. En général, on met en œuvre un pentène-3 oate d'alkyle, bien que les pentène-2 oates, les pentène-4 oates et des mélanges de penténoates d'alkyles puissent être utilisés. Dans le cadre de cette application, il s'avère préférable de choisir l'alcool (coréactif) correspondant au reste alkyle de l'ester de départ, le reste alkyle ayant avantageusement 4 atomes de carbone du maximum. De bons résultats sont obtenus au départ de l'un ou l'autre des couples de réactifs suivants : penténoate de méthyle et méthanol, penténoated'éthyle et éthanol.

En fin de réaction, ou lorsque la conversion souhaitée est atteinte on récupère l'ester linéaire

recherché par tout moyen approprié, par exemple, par distillation ou extraction liquide-liquide.

Les exemples ci-après illustrent l'invention sans toutefois en limiter le domaine ou l'esprit.

Exemples

Les conventions suivantes sont utilisées ci-après.

Dans les produits formés ne sont pas inclus les composés résultant de l'isomérisation de position de la double liaison oléfinique.

Les produits formés sont essentiellement les diesters et le pentanoate d'alkyle, ce dernier résultant de l'hydrogénation de l'ester de départ.

A désigne l'activité exprimée en moles de produits formés par heure et par atome-gramme de cobalt.

X (%) désigne le nombre de moles de diesters pour 100 moles de produits formés.

Y (%) désigne le nombre de moles d'adipate d'alkyle pour 100 moles de produits formés.

Z (%) désigne le nombre de moles de pentanoate d'alkyle pour 100 moles de produits formés.

Exemples 1 à 18 — Essais témoins (a) à (k)

On a réalisé une série d'essais selon le mode opératoire suivant :

Dans un autoclave de 125 ml en acier inoxydable, purgé sous argon on introduit du pentène-3 oate de méthyle (P3), du méthanol, du dicobaltoctacarbonyle (DCOC), de l'isoquinoléine, et le cas échéant un solvant.

L'autoclave est alors purgé par un courant de monoxyde de carbone renfermant, le cas échéant, de l'hydrogène. On porte ensuite l'autoclave à la température (T), sous une pression (P). Après un temps de réaction (désigné par t et exprimé en heures), à cette température, l'autoclave est refroidi et dégazé. Le mélange réactionnel est analysé par chromatographie en phase gazeuse. Les conditions particulières ainsi que le résultats obtenus figurent respectivement dans les tableaux I(A) et I(B) ci-après :

Dans le tableau I(A) les rapports MeOH/P3, P3(Co et N/Co désignent respectivement le rapport molaire du méthanol au pentène-3 oate, le rapport du nombre de moles de pentène-3 oate au nombre d'atomes-grammes de cobalt, et le rapport du nombre de moles d'isoquinoléine au nombre d'atomes-grammes de cobalt.

Dans le tableau I(B) :

THP désigne le tétrahydropyranne.

THT désigne le tétrahydrothiophène.

THF désigne le tétrahydrofuranne.

Me-2F désigne le méthyl-2 furanne.

DBF désigne le dibenzofuranne.

Les essais témoins (a) à (d) montrent clairement qu'en l'absence de solvant, la présence d'hydrogène se traduit par une augmentation de l'efficacité du catalyseur à base de cobalt et par une baisse notable de la sélectivité en adipate de diméthyle.

Les exemples 1 à 18 montrent que la présence simultanée d'un solvant selon l'invention et d'hydrogène permet d'obtenir sélectivement et efficacement l'adipate de diméthyle.

Exemple 19

Dans l'autoclave et selon le mode opératoire, décrits ci-avant on a réalisé un essai sur une charge constituée par :

50 mmol de pentène-2 oate de méthyle ($P_2$)
100 mmol de méthanol ($MeOH/P_2 = 2$)
0,42 mmol de dicobaltoctacarbonyle ($P_2/Co = 59,5$)
6,28 mmol d'isoquinoléine (N/Co = 7,5)
14,4 g de dioxanne-1,4 (59 % en poids).

Les résultats obtenus en deux heures de réaction à 160 °C sous 130 bars, en utilisant du monoxyde de carbone renfermant environ 2 % en volume d'hydrogène, sont les suivants :

A = 5,16
X (%) = 94,4
Y (%) = 79,0
Z (%) = 3,8

Essai témoin (m)

On reproduit l'exemple 19 en ommettant de charger le dioxanne-1,4.

Toutes conditions égales par ailleurs, les résultats obtenus sont les suivants :

A = 17,8
X (%) = 87,8
Y (%) = 63,1
Z (%) = 8,6

Exemples 20 et 21

On reproduit l'exemple 1 ci-avant en ne faisant varier que la quantité de dioxanne-1,4 chargée. Les conditions particulières ainsi que les résultats obtenus sont indiqués dans le tableau II ci-après.

Tableau II

| Exemple | Dioxanne-1,4 (g) | Dioxanne-1,4 % (poids) | A | X (%) | Y (%) | Z (%) |
|---|---|---|---|---|---|---|
| 20 | 20,8 | 67,5 | 1,55 | 96,8 | 82,7 | 2,7 |
| 1 | 10,3 | 50,7 | 3,88 | 96,7 | 83,3 | 2,8 |
| 21 | 4,1 | 29 | 4,59 | 96,2 | 80,9 | 3,3 |

Exemple 22

Dans l'autoclave et selon le mode opératoire, décrits précédemment on réalise un essai sur une charge constituée par :

50,4 mmol de pentène-3 oate de méthyle
102 mmol de méthanol ($MeOH/P_3$ = 2,02)
2,01 mmol de dicobaltoctacarbonyle ($P_3/Co$ = 12,5)
16,1 mmol de lutidine-3,4 (N/Co = 4,0)
10,3 g de dioxanne-1,4 (46 % poids).

En deux heures de réaction à 160 °C sous 130 bars, en utilisant du monoxyde de carbone renfermant environ 0,8 % en volume d'hydrogène on a obtenu les résultats suivants :

A = 2,21
X (%) = 97
Y (%) = 76,6
Z (%) = 2,7

Essais 23 à 25 — essai témoin (n)

On a réalisé une autre série d'essais selon le mode opératoire suivant :

Dans un autoclave de 300 $cm^3$ en acier inoxydable muni d'une agitation centrale à entraînement magnétique, chauffé et régulé électriquement on introduit :

300 mmol de pentène-3 oate de méthyle
600 mmol de méthanol ($MeOH/P_3$ = 2)
48 mmol de picoline-4
6 mmol de dicobaltoctacarbonyle ($P_3/Co$ = 2,5 ; N/Co = 4)

et un solvant dont la nature et la quantité sont précisées dans le tableau III ci-après.

On chauffe sous balayage d'un mélange de monoxyde de carbone, d'hydrogène et d'azote ($H_2$ = 0,22 % en volume ; $N_2$ = 5 % en volume) jusqu'à 160 °C. La pression dans l'autoclave est maintenue à 130 bars et le débit du mélange gazeux précité est de 40 l/h (CNTP).

On effectue périodiquement des prélèvements de la masse réactionnelle que l'on analyse.

Dans le tableau III ci-après on a rassemblé les résultats obtenus dans chaque essai au bout de respectivement 1 heure, 2 heures et 6 heures de réaction à la température indiquée.

Les conventions utilisées sont les mêmes que pour les exemples 1 à 22 ci-avant ; par TT (%) on entend le taux de transformation du pentène-3 oate de méthyle en produits ne résultant pas de la seule isomérisation de position de la double liaison oléfinique.

Tableau I(A)

| Réf | P3 mmol | MeOH mmol | DCOC mmol | MeOH/ P3 | P3/Co | N/Co | $H_2$ (% vol) | P bars | T °C |
|---|---|---|---|---|---|---|---|---|---|
| a | 50,1 | 109 | 1,02 | 2,17 | 24,6 | 4 | 0 | 130 | 160 |
| b | 49,7 | 99 | 0,88 | 1,99 | 28,5 | 4,5 | 0,7 | " | " |
| c | 99,8 | 198 | 2,00 | 1,98 | 25,0 | 12 | 0,9 | " | " |
| d | " | 200 | 2,01 | 2,00 | 24,8 | " | 2,6 | " | " |
| e | 50,3 | 104 | 1,00 | 2,07 | 25,2 | 3,9 | 0,8 | " | " |
| 1 | 50,0 | 102 | 1,00 | 2,04 | 25,0 | 4,0 | 0,8 | " | " |
| 2 | 50,5 | 101 | 1,00 | 2,00 | 25,3 | 4,0 | " | " | " |
| 3 | 50,0 | 102 | 0,99 | 2,04 | 25,2 | 4,3 | " | " | " |
| 4 | 50,2 | 101 | 1,00 | 2,01 | 25,1 | 4,0 | " | " | " |
| 5 | 50,0 | 102 | 1,01 | 2,04 | 24,8 | 3,9 | " | " | " |
| 6 | 50,3 | 100 | 0,99 | 1,99 | 25,4 | 4,0 | 0,9 | " | " |
| 7 | 50,2 | 100 | 1,00 | 1,99 | 25,1 | 4,0 | 1,0 | " | " |
| 8 | 50,3 | 101 | 1,01 | 2,01 | 24,9 | 4,0 | " | " | " |
| 9 | 50,2 | 98 | 1,01 | 1,95 | 24,8 | 3,9 | 0,9 | " | " |
| 10 | 50,2 | 100 | 1,00 | 1,99 | 24,8 | 4,0 | " | " | " |
| 11 | 50,5 | 101 | 1,00 | 2,00 | 25,3 | 3,8 | " | " | " |
| 12 | 50 | 101 | 1,00 | 2,02 | 25,0 | 4,0 | " | " | " |
| f | 49,6 | 104 | 0,98 | 2,09 | 25,0 | 20,6 | 0,8 | " | " |
| 13 | 49,7 | 102 | 1,00 | 2,05 | 25,0 | 20,0 | " | " | " |
| g | 100 | 198 | 1,98 | 1,98 | 25,4 | 0,53 | " | " | " |
| 14 | 100 | 202 | 2,00 | 2,02 | 25,1 | 0,54 | " | " | " |
| h | 49,7 | 100 | 1,03 | 2,01 | 24,1 | 3,8 | " | " | 140 |
| 15 | 50,1 | 101 | 0,98 | 2,01 | 25,5 | 4,1 | " | " | " |
| i | 100 | 198 | 3,90 | 1,98 | 12,7 | 4,0 | " | " | 160 |
| 16 | 50 | 102 | 2,01 | 2,04 | 12,4 | " | " | " | " |
| j | 100 | 200 | 2 | 2 | 25 | 2 | 0,4 | 80 | " |
| 17 | 50 | 100 | 1 | " | " | " | " | " | " |
| h | 100 | 200 | 2 | " | " | 12 | 1 | 200 | " |
| 18 | 50 | 100 | 1 | " | " | " | " | " | " |

Tableau I(B)

| Réf. | SOLVANT nature | SOLVANT (% pds) | t | A | X (%) | Y (%) | Z (%) |
|---|---|---|---|---|---|---|---|
| a | - | 0 | 1 | 3,7 | 95,1 | 79,4 | 4,9 |
| b | - | 0 | " | 10,4 | 89,0 | 74,6 | 10,4 |
| c | - | 0 | " | 1,9 | 94,7 | 75,5 | 4,9 |
| d | - | 0 | " | 3,3 | 92,5 | 74,7 | 6,8 |
| e | - | 0 | 2 | 7,9 | 92,3 | 76,4 | 7,0 |
| 1 | dioxanne-1,4 | 50 | 2 | 3,9 | 96,7 | 83,3 | 2,8 |
| 2 | dithianne-1,4 | 50 | 5 | 2,5 | 95,2 | 83,6 | 4,4 |
| 3 | trithianne-1,3,5 | 50 | 2 | 2,9 | 94,1 | 80,2 | 5,9 |
| 4 | trioxanne-1,3,5 | 50 | " | 3,8 | 95,9 | 79,2 | 3,7 |
| 5 | THP | 50 | " | 4,8 | 93,8 | 80,7 | 5,4 |
| 6 | thianthrène | 50 | " | 7,7 | 94,2 | 79,4 | 5,2 |
| 7 | THT | 50 | " | 4,2 | 95,7 | 84,0 | 3,6 |
| 8 | benzothiazole | 50 | " | 1,9 | 95,0 | 80,7 | 4,8 |
| 9 | THF | 50 | " | 4,1 | 95,2 | 81,2 | 4,4 |
| 10 | thiophène | 54 | " | 2,0 | 94,7 | 82,2 | 4,6 |
| 11 | Me-2F | 50 | " | 4,8 | 94,7 | 82,1 | 4,6 |
| 12 | DBF | 50 | " | 5,6 | 95,3 | 80,6 | 4,1 |
| f | - | 0 | " | 1,1 | 90,1 | 73,7 | 8,8 |
| 13 | dioxanne-1,4 | 43 | " | 3,1 | 94,2 | 77,0 | 5,8 |
| g | - | 0 | " | 3,2 | 92,8 | 73,2 | 5,6 |
| 14 | dioxanne-1,4 | 37 | " | 1,9 | 92,4 | 74 | 5,4 |
| h | - | 0 | " | 3,4 | 94,0 | 70,3 | 5,6 |
| 15 | dioxanne-1,4 | 50 | " | 1,1 | 95,8 | 75,8 | 3,5 |
| i | - | 0 | " | 3,9 | 91,3 | 73,1 | 8,4 |
| 16 | dioxanne-1,4 | 50 | " | 2,0 | 92,6 | 78,2 | 6,8 |
| j | - | 0 | " | 3,4 | 84,2 | 70,2 | 15,3 |
| 17 | THT | 50 | " | 1,1 | 91,9 | 78,4 | 7,5 |
| k | - | 0 | " | 2,4 | 96,0 | 68 | 3,4 |
| 18 | THT | 50 | " | 2,9 | 97,1 | 80,6 | 2,5 |

Tableau (II)

| Réf | SOLVANT | Durée en heure | TT (%) | X (%) | Y (%) | Z (%) |
|---|---|---|---|---|---|---|
| n | méthylisobutylcétone | 1 | 29,8 | 92,5 | 78,2 | 7,0 |
| | 60 ml (44,6 % poids) | 2 | 53,3 | 92,6 | " | 6,9 |
| | | 6 | 97,1 | 92,2 | 77,8 | 7,4 |
| 23 | dioxanne-1,4 | 1 | 23,2 | 92,8 | 79,0 | 6,8 |
| | 60 ml (50 % poids) | 2 heures 20 mn | 47,4 | 93,1 | 79,8 | 6,4 |
| | | 6 | 93,6 | 92,2 | 79,2 | 6,7 |
| 24 | thiphène | 1 | 16,6 | 93,9 | 80,8 | 5,8 |
| | 60 ml (51 % poids) | 2 heures 10 mn | 31,3 | 94,2 | 81,2 | 5,3 |
| | | 6 | 72,5 | 94,1 | 80,6 | 5,4 |
| 25 | tétrahydrotiophène | 1 | 18,8 | 95,3 | 83,9 | 4,3 |
| | 60 ml (49 % poids) | 2 | 36,1 | 95,2 | 83,5 | 4,4 |
| | | 6 | 84,7 | 95,1 | 83,3 | 4,4 |

**Revendications**

1. Procédé de préparation d'esters linéaires saturés par réaction d'un alcool de formule R—OH et du monoxyde de carbone sur un composé renfermant une liaison oléfinique unique de formule $R_1CH = CHR_2$ dans lesquelles :

$R_1$ et $R_2$, identiques ou différents, représentent l'hydrogène ou un radical alkyle ayant au maximum 20 atomes de carbone, pouvant être substitué par 1 ou 2 atomes de chlore ou groupes alcoxy renfermant au maximum 4 atomes de carbone,

$R_1$ pouvant en outre représenter un radical $-(CH_2)_p-COOH$, $-(CH_2)_p-COOR_3$ ou $-(CH_2)_p-CN$ dans lequel p est un entier au plus égal à 6 pouvant être nul, $R_3$ représente un radical alkyle comportant au maximum 12 atomes de carbone, un à deux groupements méthylène pouvant en outre comporter un substituant alkyle ayant au maximum 4 atomes de carbone,

$R_1$ et R2 pouvant en outre former ensemble un radical unique bivalent $-(CH_2)_q-$, comportant le cas échéant un ou deux substituants alkyle ayant au maximum 4 atomes de carbone, q étant un entier compris entre 3 et 6 inclus,

et R est un radical alkyle comportant au maximum 12 atomes de carbone éventuellement substitué par un ou deux groupes hydroxyles, un radical cycloalkyle ayant de 5 à 7 atomes de carbone, un radical aralkyle ayant de 7 à 12 atomes de carbone ou un radical phényle, en présence de cobalt, d'une base azotée tertiaire et, le cas échéant d'hydrogène, caractérisé en ce que la réaction est conduite dans un solvant choisi parmi les composés hétérocycliques renfermant un seul hétérocycle ; ledit hétérocycle, formé de 5 à 6 chaînons, contient de 1 à 3 hétéroatomes non adjacents, identiques ou différents et, choisis parmi l'oxygène et le soufre divalent et peut comporter, le cas échéant, une ou deux doubles liaisons carbone-carbone ; un cycle à 5 chaînons pouvant comporter en outre un hétéroatome d'azote, séparé d'un hétéroatome d'oxygène ou de soufre par au moins un atome de carbone du cycle et relié à l'un des atomes de carbone adjacent par une double liaison.

2. Procédé selon la revendication 1, où dans le composé de formule $R_1CH = CHR_2$, $R_1$ et $R_2$, identiques ou différents représentent l'hydrogène ou un radical alkyle ayant au maximum 10 atomes de carbone, ou bien forment ensemble un radical unique bivalent $-(CH_2)_q-$ q ayant la signification donnée dans la revendication 1, ledit radical pouvant comporter le cas échéant 1 ou 2 substituants méthyle.

3. Procédé selon la revendication 1, où dans le composé de formule $R_1CH = CHR_2$, $R_1$ représente un radical $-(CH_2)_p-COOR_3$, p et $R_3$ ayant la signification donnée dans la revendication 1, un à deux groupements méthylène pouvant comporter un substituant alkyle ayant au maximym 4 atomes de carbone, et $R_2$ représente l'hydrogène ou un radical alkyle ayant au maximum 4 atomes de carbone.

4. Procédé selon la revendication 3, caractérisé en ce que le composé renfermant une liaison oléfinique unique est un penténoate d'alkyle.

5. Procédé selon l'une quelconque des revendications précédentes, caractérisé en ce que le solvant est choisi parmi lesdits composés hétérocycliques qui renferment exclusivement un (ou plusieurs) hétéroatome(s) de soufre.

6. Procédé selon l'une quelconque des revendications 1 à 4, caractérisé en ce que le solvant est choisi parmi lesdits composés hétérocycliques qui comportent 6 chaînons et au moins 2 hétéroatomes de même nature et choisis parmi l'oxygène et le soufre divalent.

7. Procédé selon l'une quelconque des revendications 1 à 4, caractérisé en ce que le solvant est choisi dans le groupe constitué par le thiophène, le tétrahydrothiophène, le furanne, le tétrahydrofuranne, le dioxanne-1,4, le dithiane-1,è et le trithiane.

8. Procédé selon l'une quelconque des revendications précédentes, caractérisé en ce que le solvant représente au moins 20 % en poids du mélange réactionnel initiale.

9. Procédé selon la revendication 8, caractérisé en ce que le solvant représente de 30 à 60 % en poids du mélange réactionnel initial.

10. Procédé selon l'une quelconque des revendications précédentes, caractérisé en ce que l'hydrogène représente au maximum 3 % (en volume) du monoxyde de carbone.

11. Procédé selon l'une quelconque des revendications précédentes, caractérisé en ce que le rapport atomique N/Co est compris entre 1 et 50 et, de préférence entre 2 et 25.

12. Procédé selon l'une quelconque des revendications précédentes, caractérisé en ce que la température de réaction est comprise entre 120 et 200 °C, de préférence, entre 130 et 180 °C.

13. Procédé selon l'une quelconque des revendications précédentes, caractérisé en ce que la pression est comprise entre 50 et 1 000 bars et, de préférence, entre 100 et 300 bars.

**Claims**

1. Process for preparing saturated linear esters by reacting an alcohol of formula R—OH and carbon monoxide with a compound containing a single olefinic bond of formula $R_1CH = CHR_2$ in which :

$R_1$ and $R_2$, which may be identical or different, denote hydrogen or an alkyl radical which has a maximum of 20 carbon atoms, and can be substituted with 1 or 2 chlorine atoms or alkoxy groups containing a maximum of 4 carbon atoms,

$R_1$ being able, in addition, to denote a $-(CH_2)_p-COOH$, $-(CH_2)_p-COOR_3$ or $-(CH_2)_p-CN$ radical in which p is an integer at most equal to 6 and which can be zero, and $R_3$ denotes an alkyl radical containing a maximum of 12 carbon atoms, one to two methylene groups being able, in addition, to bear an alkyl substituent having a maximum of 4 carbon atoms,

$R_1$ and $R_2$ being able, in addition, to form together a single bivalent radical $-(CH_2)_q-$ containing, where appropriate, one or two alkyl substituents having a maximum of 4 carbon atoms, q being an integer between 3 and 6 inclusive,

and R is an alkyl radical containing a maximum of 12 carbon atoms and optionally substituted with one or two hydroxyl groups, a cycloalkyl radical having from 5 to 7 carbon atoms, an aralkyl radical having from 7 to 12 carbon atoms or a phenyl radical, in the presence of cobalt, a tertiary nitrogenous base and, where appropriate, hydrogen, characterised in that the reaction is performed in a solvent chosen from heterocyclic compounds containing a single heterocyclic system ; the said heterocyclic system, composed of 5 or 6 members, contains from 1 to 3 non-adjacent hetero atoms, which may be identical or different, chosen from oxygen and divalent sulphur, and can contain, where appropriate, one or two carbon-carbon double bonds ; a 5-membered ring being able to contain, in addition, a nitrogen hetero atom, separated from an oxygen or sulphur heteroatom by at least one carbon atom of the ring and connected to one of the adjacent carbon atoms by a double bond.

2. Process according to Claim 1, in which, in the compound of formula $R_1CH = CHR_2$, $R_1$ and $R_2$, which may be identical or different, denote hydrogen or an alkyl radical having a maximum of 10 carbon atoms, or alternatively form together a single bivalent radical $-(CH_2)_q-$, q having the significance given in Claim 1, the said radical being able to contain, where appropriate, 1 or 2 methyl substituents.

3. Process according to Claim 1, in which, in the compound of formula $R_1CH = CHR_2$, $R_1$ denotes a $-(CH_2)_p-COOR_3$ radical, p and $R_3$ having the significance given in Claim 1, and one to two methylene groups can contain an alkyl substituent having a maximum of 4 carbon atoms,

and $R_2$ denotes hydrogen or an alkyl radical having a maximum of 4 carbon atoms.

4. Process according to Claim 3, characterised in that the compound containing a single olefinic bond is an alkyl pentenoate.

5. Process according to any one of the preceding claims, characterised in that the solvent is chosen from the said heterocyclic compounds which exclusively contain one (or more) hetero atom(s) of sulphur.

6. Process according to any one of Claims 1 to 4, characterised in that the solvent is chosen from the said heterocyclic compounds which contain 6 members and at least 2 hetero atoms of the same nature, chosen from oxygen and divalent sulphur.

7. Process according to any one of Claims 1 to 4, characterised in that the solvent is chosen from the group consisting of thiophene, tetrahydrothiophene, furan, tetrahydrofuran, 1,4-dioxane, 1,4-dithiane and trithiane.

8. Process according to any one of the preceding claims, characterised in that the solvent represents at least 20 % by weight of the initial reaction mixture.

9. Process according to Claim 8, characterised in that the solvent represents from 30 to 60 % by weight of the initial reaction mixture.

10. Process according to any one of the preceding claims, characterised in that hydrogen represents a maximum of 3 % (by volume) of the carbon monoxide.

11. Process according to any one of the preceding claims, characterised in that the atomic ratio N/Co is between 1 and 50, and preferably between 2 and 25.

12. Process according to any one of the preceding claims, characterised in that the reaction temperature is between 120 and 200 °C, and preferably between 130 and 180 °C.

13. Process according to any one of the preceding claims, characterised in that the pressure is between 50 and 1 000 bars, and preferably between 100 and 300 bars.

**Patentansprüche**

1. Verfahren zur Herstellung von gesättigten linearen Estern durch Umsetzung eine Alkohols der Formel R—OH und von Kohlenmonoxid mit einer Verbindung, die eine einzige olefinische Bindung enthält, der Formel $R_1CH = CHR_2$, in der :

$R_1$ und $R_2$ gleich oder verschieden sind und (jeweils) für Wasserstoff oder eine Alkylgruppe mit maximal 20 Kohlenstoffatomen stehen, die mit 1 oder 2 Chloratomen oder Alkoxygruppen, die maximal 4 Kohlenstoffatome enthalten, substituiert sein kann,

$R_1$ außerdem eine Gruppe $-(CH_2)_p-COOH$, $-(CH_2)_p-COOR_3$ oder $-(CH_2)_p-CN$ sein kann, in der p eine ganze Zahl von maximal 6 ist und 0 sein kann, $R_3$ eine Alkylgruppe mit maximal 12 Kohlenstoffatomen bedeutet (oder) 1 oder 2 Methylengruppen, die außerdem einen Alkylsubstituenten mit maximal 4 Kohlenstoffatomen tragen können,

$R_1$ und $R_2$ außerdem zusammen eine einzige zweiwertige Gruppe $-(CH_2)_q-$ bilden können, die gegebenenfalls 1 oder 2 Alkylsubstituenten mit maximal 4 Kohlenstoffatomen trägt, wobei q eine ganze Zahl von 3 bis 6 einschließlich ist, und (wobei)

R eine Alkylgruppe mit maximal 12 Kohlenstoffatomen ist, gegebenenfalls substituiert mit 1 oder 2

Hydroxylgruppen, eine Cycloalkylgruppe mit 5 bis 7 Kohlenstoffatomen, eine Aralkylgruppe mit 7 bis 12 Kohlenstoffatomen oder eine Phenylgruppe, in Gegenwart von Cobalt, einer tertiären Stickstoffbase und gegebenenfalls Wasserstoff, dadurch gekennzeichnet, daß die Umsetzung in einem Lösungsmittel ausgewählt unter heterocyclischen Verbindungen mit einem einzigen Heterocyclus vorgenommen wird, wobei der aus 5 oder 6 Ringgliedern gebildete Heterocyclus 1 bis 3 nicht benachbarte Heteroatome enthält, die gleich oder verschieden sind und unter Sauerstoff und zweiwertigem Schwefel ausgewählt sind, und gegebenenfalls 1 oder 2 Kohlenstoff-Kohlenstoff-Doppelbindungen enthalten kann, (und) wobei ein 5-gliedriger Ring zusätzlich ein Stickstoff-Heteroatom enthalten kann, das von einem Sauerstoff- oder Schwefel-Heteroatom durch mindestens ein Ring-Kohlenstoffatom getrennt und an eines der benachbarten Kohlenstoffatome durch eine Doppelbindung gebunden ist.

2. Verfahren nach Anspruch 1, bei dem in der Verbindung der Formel $R_1CH = CHR_2$ $R_1$ und $R_2$, die gleich oder verschieden sind, Wasserstoff oder eine Alkylgruppe mit maximal 10 Kohlenstoffatomen bedeuten oder zusammen eine einzige zweiwertige Gruppe $-(CH_2)_q-$ bilden, wobei q die in Anspruch 1 gegebene Bedeutung hat und diese Gruppe gegebenenfalls 1 oder 2 Methylgruppen tragen kann.

3. Verfahren nach Anspruch 1, bei dem in der Verbindung der Formel $R_1CH = CHR_2$ $R_1$ eine Gruppe $-(CH_2)_p-COOR_3$, wobei p und $R_3$ die in Anspruch 1 angegebene Bedeutung haben (oder) 1 oder 2 Methylengruppen, die eine Alkylgruppe mit maximal 5 Kohlenstoffatomen tragen kann, bedeutet, und $R_2$ für Wasserstoff oder eine Alkylgruppe mit maximal 4 Kohlenstoffatomen steht.

4. Verfahren nach Anspruch 3, dadurch gekennzeichnet, daß die Verbindung, die eine einzige olefinische (Doppel) Bindung enthält, ein Alkylpentenoat ist.

5. Verfahren nach einem der vorangehenden Ansprüche, dadurch gekennzeichnet, daß das Lösungsmittel unter den genannten heterocyclischen Verbindungen ausgewählt wird, die ausschließlich ein (oder mehrere) Schwefelheteroatom(e) enthalten.

6. Verfahren nach einem der Ansprüche 1 bis 4, dadurch gekennzeichnet, daß das Lösungsmittel unter den heterocyclischen Verbindungen ausgewählt wird, die 6 Ringglieder umfassen und mindestens 2 Heteroatome gleicher Beschaffenheit, ausgewählt unter Sauerstoff und zweiwertigem Schwefel.

7. Verfahren nach einem der Ansprüche 1 bis 4, dadurch gekennzeichnet, daß das Lösungsmittel ausgewählt wird aus der Gruppe bestehend aus Thiophen, Tetrahydrothiophen, Furan, Tetrahydrofuran, 1,4-Dioxan, 1,4-Dithian und Trithian.

8. Verfahren nach einem der vorangehenden Ansprüche, dadurch gekennzeichnet, daß das Lösungsmittel mindestens 20 Gew.-% des ursprünglichen Reaktionsgemisches ausmacht.

9. Verfahren nach Anspruch 8, dadurch gekennzeichnet, daß das Lösungsmittel 30 bis 60 Gew.-% des ursprünglichen Reaktionsgemisches ausmacht.

10. Verfahren nach einem der vorangehenden Ansprüche, dadurch gekennzeichnet, daß der Wasserstoff maximal 3 Vol.-% des Kohlenmonoxids ausmacht.

11. Verfahren nach einem der vorangehenden Ansprüche, dadurch gekennzeichnet, daß das Atomverhältnis N/Co 1 bis 50, vorzugsweise 2 bis 25 beträgt.

12. Verfahren nach einem der vorangehenden Ansprüche, dadurch gekennzeichnet, daß die Reaktionstemperatur im Bereich von 120 bis 200 °C, vorzugsweise von 130 bis 180 °C liegt.

13. Verfahren nach einem der vorangehenden Ansprüche, dadurch gekennzeichnet, daß der Druck 50 bis 1 000 bar und vorzugsweise 100 bis 300 bar beträgt.